Europäisches Patentamt

European Patent Office

Office européen des brevets

(11) Publication number: **0 081 907**
**B1**

(12) **EUROPEAN PATENT SPECIFICATION**

(45) Date of publication of patent specification: **28.05.86**

(21) Application number: **82305995.1**

(22) Date of filing: **11.11.82**

(51) Int. Cl.⁴: **A 61 F 5/44**

(54) **Sealing means for ostomy appliances.**

(30) Priority: **27.11.81 US 325465**

(43) Date of publication of application:
**22.06.83 Bulletin 83/25**

(45) Publication of the grant of the patent:
**28.05.86 Bulletin 86/22**

(84) Designated Contracting States:
**AT BE CH DE FR GB IT LI LU NL SE**

(56) References cited:
**EP-A-0 024 253**
**CH-A- 531 353**
**GB-A- 839 818**
**US-A-3 712 304**
**US-A-3 804 091**
**US-A-3 814 648**

(73) Proprietor: **E.R. Squibb & Sons, Inc.**
**Lawrenceville-Princeton Road**
**Princeton, N.J. 08540 (US)**

(72) Inventor: **Cilenta, Rudolfo D.**
**478 Hobart St.**
**North Brunswick New Jersey (US)**
Inventor: **Smith, Edward C., Jr.**
**1006 Hunters Glen**
**Plainsboro New Jersey (US)**
Inventor: **Freeman, Frank M.**
**10 Brandon Road**
**Lawrenceville New Jersey (US)**

(74) Representative: **Thomas, Roger Tamlyn et al**
**D. Young & Co. 10 Staple Inn**
**London WC1V 7RD (GB)**

EP 0 081 907 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to assemblies for attachment to the skin around a stoma, otherwise known as skin barriers.

Major abdominal surgery for a number of diseases involving different parts of the gastro-intestinal and urinary tract can result in a patient being left with an abdominal stoma. The three most common types of abdominal stoma are the colostomy, the ileostomy, and the ileal conduit. In the case of an ileostomy, ileal conduit, and many colostomy operations, the patient is unable to control the passage of bodily waste material and must rely upon an appliance attached to their body to collect this material.

Numerous appliances have been proposed for this purpose. Most can be characterized as either a one-piece or a two-piece system. The one-piece appliance conventionally consists of a pouch having an opening in one sidewall for the stoma around which a plastic faceplate is permanently bonded. The faceplate includes an outer layer of adhesive material which is designed to affix the appliance directly to the body or to an intermediate skin barrier or sealing washer. The two-piece appliance conventionally consists of a mounting ring that is supported on the body by means of an elastic belt.

Recently, a two-piece appliance disclosed by Steer et al. in British Patent 1 571 657 has achieved considerable commercial success. The Steer et al. appliance consists of a skin barrier having a projecting rib type coupling member affixed to its outer surface and a pouch with a channel shaped coupling member encircling the stoma opening in the pouch sidewall. The pouch can be securely attached to the skin barrier by snapping onto the rib. The skin barrier employed by Steer et al. uses the material described by Chen in U.S. Patent 3 339 546 and includes an adhesive layer consisting of a mixture of gelatin, pectin, sodium carboxymethyl-cellulose, and polyisobutylene and an outer water insoluble polyethylene film to which the rib coupling member is affixed.

This invention is directed to a composite type skin barrier product which includes a coupling element to which a pouch can be easily and securely attached.

According to one aspect of the invention, there is provided an assembly for attachment to the skin around a stoma, which comprises a first component (A) comprising an adhesive layer and an upper polymeric film said first component having a centrally located stomal opening, a second component (B) comprising a coupling element including an outwardly extending flange wherein said flange is permanently affixed to the polymeric film of said first component (A) in an area surrounding said stomal opening, and a third component (C) comprising a microporous adhesive layer and an upper porous backing layer said microporous adhesive layer overlapping said coupling element flange and polymeric film and extending beyond the borders of said first component.

According to another aspect of the invention, there is provided an assembly for attachment to the skin around a stoma, which comprises a first component (A) comprising an adhesive layer and an upper polymeric film said first component having a centrally located stomal opening, an intermediate component (C) comprising a microporous adhesive layer and an upper porous backing layer said intermediate layer being bonded to and covering at least a portion of the outer polymeric film of said first layer and wherein said intermediate component (C) extends beyond the borders of said first component (A) and a coupling component (B) including an outwardly extending flange wherein said flange is permanently affixed to said porous backing layer in an area surrounding said stomal opening.

The skin barrier hereinafter described has improved flexibility and is lighter and more comfortable on the body than those currently employed. As a result of its increased flexibility, it provides a stronger bond with the skin and is not subject to cracking, creasing, or lifting as the anatomy underneath bends or stretches. It includes a first adhesive component which fits around a stoma, a coupling element, and a second ahesive component which is flexible, light, and a degree of porosity or breathability.

In one embodiment, the coupling element is permanently affixed to the top of the first adhesive component around the stomal opening and the second adhesive component overlaps a flange portion of the coupling element and any exposed top portion of the first adhesive component which is exterior to the coupling element.

In an alternative embodiment, the porous adhesive component is bonded to the top of the first adhesive component and the coupling element is affixed directly to the second adhesive component.

Preferred embodiments of the invention will now be described by way of example with reference to the drawings in which:—

Figure 1 is a top view of a first embodiment of the composite skin barrier of this invention including the preferred concentrically shaped coupling element.

Figure 2 is a front view taken along line 2—2 of Figure 1.

Figure 3 is an exploded view of the composite skin barrier as shown in Figure 2 in greatly enlarged detail.

Figure 4 is a front view of an ostomy pouch having a coupling element enabling it to be affixed to the skin barrier shown in Figures 1 to 3.

Figure 5 is an enlarged section taken in a vertical plane of the pouch coupling element.

Figure 6 is an exploded view similar to Figure 3 of an alternative embodiment of the composite skin barrier of this invention.

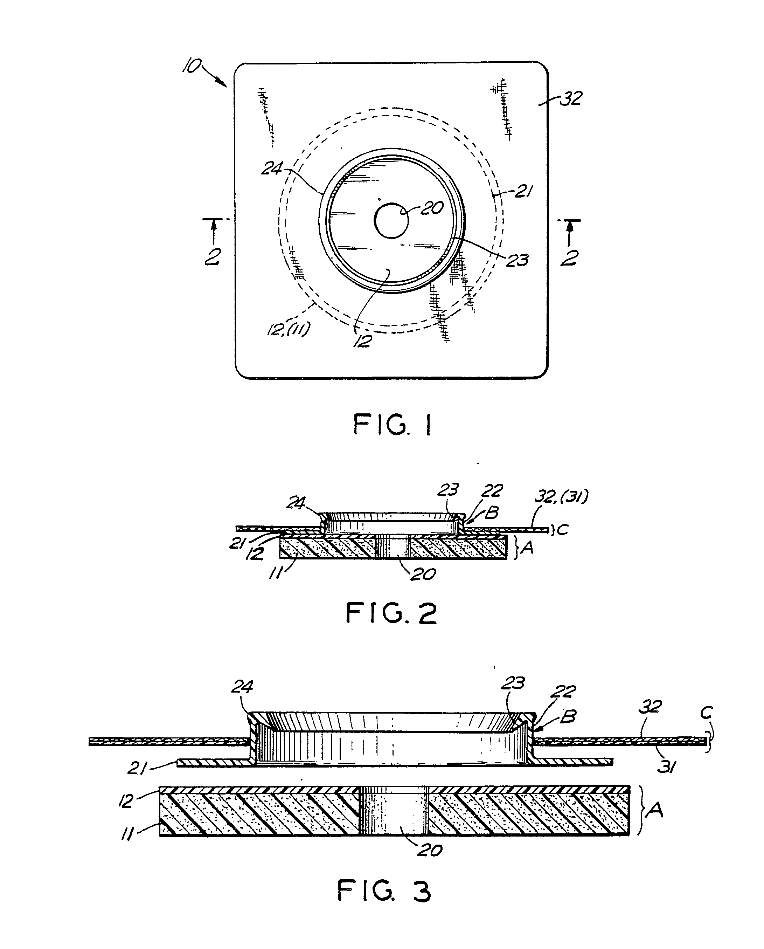

Referring to Figures 1—3 in more particular detail, the composite skin barrier 10 consists of three components. Component A includes an adhesive layer 11 and an outer polymeric film 12 and is shown with a centrally located opening or starter hole 20 which can be enlarged to fit snugly around a stoma. Component B is a coupling element which is permanently affixed to outer polymeric film 12. Component C is a flexible microporous tape having an adhesive layer 31 that overlaps flange portion 21 of the coupling element and any of polymeric film 12 not affixed to flange 21. Outer surface 32 of the microporous tape is a porous backing layer. In use, adhesive surface 31 is pressed onto the body a distance from the stoma and aids adhesive layer 11 in maintaining the skin barrier in place. Component A may extend exactly to the end of flange 21 of component B or slightly beyond as shown in Figures 1 to 3.

The adhesive layer 11 is a homogeneous blend of one or more pressure sensitive adhesive materials having intimately dispersed therein one or more water soluble hydrocolloid gums. Optionally, one or more thermoplastic elastomers can be included with the pressure sensitive adhesive materials and one or more water swellable or inert cohesive strengthening agents can be included with the hydrocolloid gums.

Suitable pressure sensitive adhesive materials for inclusion in adhesive layer 11 are various natural or synthetic viscous or elastomeric substances such as natural rubber, silicone rubber, acrylonitrile rubber, polyurethane rubber, polyisobutylene, etc., either possessing dry tack by themselves or developing such tack upon the addition of a plasticizer. Low molecular weight polyisobutylenes having a viscosity average molecular weight of from about 36,000 to about 58,000 (Florey) possessing pressure sensitive adhesive properties are preferred. Such polyisobutylenes are commercially available under the trademark Vistanex from Exxon as grades LM-MS and LM-MH. One or more thermoplastic elastomers can optionally be included with the pressure sensitive adhesive substances in order to impart the properties of rubber-like extensibility and both rapid and complete recovery from modular strains. Suitable thermoplastic elastomers include medium molecular weight polyisobutylenes having a viscosity average molecular weight of from about 1,150,000 to 1,600,000 (Florey), butyl rubber which is a copolymer of isobutylene with a minor amount of isoprene having a viscosity average molecular weight of from about 300,000 to about 450,000 (Florey), and styrene copolymers such as styrene-butadiene-styrene (S-B-S), styrene-isoprene-styrene (S-I-S), and styrene-ethylene/butylene-styrene (S-EB-S) which are commercially available, for example, from Shell Chemical Co. under the trademark Kraton as Kraton D1100, Kraton D1107, Kraton 4000, Kraton G1600, and Kraton G4600. Preferred thermoplastic elastomers are butyl rubber having a viscosity average molecular weight of about 425,000 (commercially available as grade 077), polyisobutylene having a viscosity average molecular weight of about 1,200,000 (commercially available under the trademark Vistanex from Exxon as grade L-100), and styrene-isoprene-styrene (S-I-S) copolymers (commercially available from Shell as Kraton D1107).

The pressure sensitive adhesive component including the optional thermoplastic elastomer is present at from about 30% to about 70% by weight of adhesive layer 11, preferably from about 40% to about 50% by weight. The thermoplastic elastomer can be employed at up to three times the weight of the pressure sensitive elastomeric substances but preferably the thermoplastic elastomer if present will be at from about 20% to about 40% by weight of the pressure sensitive elastomeric substance.

Suitable water soluble hydrocolloids for inclusion in adhesive layer 11 are sodium carboxymethylcellulose, pectin, gelatin, guar gum, locust bean gum, and gum karaya. These gums impart wet tack, i.e., the ability to adhere to moist surfaces, to adhesive layer 11. One or more water swellable cohesive strengthening agents can optionally be included with the water soluble hydrocolloids in order to control the rate of hydration of water soluble gums. Suitable cohesive strengthening agents include finely divided substantially water insoluble cross-linked sodium carboxymethylcellulose, finely divided substantially water insoluble starch-acrylonitrile graft copolymer such as that described in U.S. Patent 3,661,815, finely divided substantially water insoluble cross-linked dextran such as that commercially available under the trademark Sephadex, finely divided purified wood cellulose such as that commercially available under the trademark Solka-Floc, and finely divided inert natural or synthetic fibrous material such as cotton.

The water soluble hydrocolloids and the optional water swellable or inert cohesive strenghtening agents together are present at from about 35% to about 65% by weight of adhesive layer 11, preferably from about 45% to about 60% by weight.

Polymeric film 12 is laminated onto the surface of adhesive layer 11 and is a thin continuous or discontinuous film of polymeric material such as polyethylene, polypropylene, polyurethane, polyvinylchloride, etc. In component A, the adhesive layer 11 will vary in thickness from about 10 to about 120 mils (about 254 to about 3048 microns) and the film 12 will vary in thickness from about 1 to about 10 mils (about 25.4 to about 254 microns).

Small amounts, i.e., less than about 10% by weight of the adhesive layer 11, of other ingredients may be included in the adhesive layer 11. For example, a plasticizer such as mineral oil, and antioxidant such as butylated hydroxyanisole, a deodorant or perfume agent may be included. In addition, small amounts of a pharmacologically active ingredient can be included in the adhesive composition. For example, an antibiotic or antimicrobial agent such as neomysin, an antiseptic agent such as povidone iodine, or antiinflammatory agent such as hydrocortisone or triamcinolone acetonide.

Component A is prepared as follows. A premix is prepared of the water soluble gums, water swellable or inert cohesive strengthening agent and any other optional substances. The premixed powder is then placed in a heavy duty high shear sigma blade or equivalent type mixer. The viscous pressure sensitive adhesive component is then added in two or three equal segments. Mixing is allowed to proceed for approximately ten minutes between each addition of the viscous material. The resultant dough-like mass is then extruded or rolled or pressed to desired thickness. In working with large batches of material, the dough-like mass may be kneaded prior to the extrusion step. Alternatively, the process may be varied by first working the viscous pressure sensitive adhesive material in the mixer for about ten minutes and then adding the powder premix to two or three equal segments with agitation for about 15 minutes between each addition. When the pressure sensitive adhesive component includes an amount of optional thermoplastic elastomer, such elastomer is first blended by geometric dilution with the pressure sensitive adhesive material in a heated high shear sigma blade or equivalent type mixer. Polymeric film 12 is then laminated onto one side of the adhesive layer and silicone coated release paper on the other to form large slabs or a continuous web of the adhesive laminate. Component A of desired configuration including starter hole 20 are then die cut.

Component B is a coupling element. Preferably, the coupling element (as best shown in Figure 3) is in the form of a cylindrical rib 22 extending substantially perpendicularly from a flat flange 21 and includes a thin resilient flexible and deflectible seal strip 23. As shown, the seal strip 23 is of tapering form seen in cross-section and extends at an angle radially inwardly from an inner surface of rib 22. Another surface of rib 22 may be provided as shown with a peripheral rim 24. Coupling B is formed from any suitable polymeric material such as polyethylene, polypropylene, etc., and is permanently affixed to the surface of polymeric film 12 by heat sealing, ultrasonic welding, by impulse welding, or by use of adhesives. Of course, coupling element B is affixed to film 12 so as to surround starter hole 20.

Component C consists of microporous adhesive layer 31 and a porous backing layer 32. The term microporous is used since the surface of adhesive layer 31 appears to be continuous but when viewed under a microscope the adhesive layer is revealed to be sponge-like having randomly located channels and voids.

The microporous adhesive layer can be of the acrylic type as taught by Copeland in U.S. Patent 3,121,021. However, preferably, the adhesive layer 31 is made of the same ingredients as adhesive layer 11 with the microporosity resulting from a difference in the manner of processing. Thus, adhesive layer 31 is preferably a homogeneous blend of one or more pressure sensitive viscous or elastomeric materials having intimately dispersed therein one or more water soluble hydrocolloid gums and optionally including one or more thermoplastic elastomers and/or one or more water swellable cohesive strengthening agents. In addition to the various minor optional ingredients such as antioxidants, preservatives, plasticizers, etc., which can be incorporated in either adhesive layer 11 and 31, adhesive layer 31 also can include up to 25% by weight of a tackifier such as terpene resin.

Preferably, adhesive layer 31 includes as the pressure sensitive adhesive and thermoplastic elastomer a mixture of a low molecular weight polyisobutylene (grade LM-MH or LM-MS) and a medium molecular weight polyisobutylene (grade L-100). Such elastomeric materials comprise from about 30% to about 60% by weight of adhesive layer 31, preferably from about 35% to about 50% by weight of adhesive layer 31. The water soluble hydrocolloids and optional water swellable cohesive strengthening agents are present at from about 20% to about 65% by weight of adhesive layer 31, preferably from about 30% to about 60% by weight of adhesive layer 31. Adhesive layer 31 also preferably includes as a plasticizer up to 10% by weight of mineral oil and up to 25% by weight of terpene resin.

The adhesive layer 31 varies from about 1 mil to about 10 mils (about 25.4 to about 254 microns) in thickness and contains holes or pores of from about 10 microns to about 300 microns in size and a porosity of about 1 to about 100 cc/sec/in$^2$ (about 0.155 to about 15.5 cc/sec/cm$^2$) as determined by ASTM D-726-71 method using Gurley Densometer 4110 at 4.89 inches (12.4 cm) of water $\Delta P$.

Porous backing layer 32 can be formed of woven or non-woven fabric such as the rayon web described by Copeland in U.S. Patent 3,121,021, an open mesh polymeric substance such as an open mesh polyethylene or polypropylene or a polymeric foam such as polyurethane foam, polyethylene foam, etc., or a non-woven material made from polyester fibers, polypropylene fibers, nylon fibers, composite olefin fibers, or cellulose fibers which are commercially available.

These non-woven spun bonded materials are the preferred porous backing layers. The porous backing layer can vary in thickness from about 3 mils to about 20 mils (about 76 to about 508 microns).

Component C is prepared according to the procedure of U.S. Patent 3,121,021 when adhesive layer 31 is an acrylic type adhesive. When adhesive layer 31 is a mixture of elastomeric substances and hydrocolloids, then component C is prepared by dispersing the viscous or elastomeric pressure sensitive adhesive materials, thermoplastic elastomers, water soluble hydrocolloids, water swellable cohesive strengthening agents, tackifiers, plasticizers, and other optional ingredients in a hydrocarbon solvent such as toluene, heptane, or hexane or mixtures thereof to form a slurry. The slurry is then deposited, for example, by means of a knife-over-roller, onto a web of silicone coated release paper. The slurry is deposited at a wet thickness of from about 5 mils to about 40 mils (about 127 to about 1016 microns), preferably about 10 mils (about 254 microns) thick. The release paper having the adhesive layer 31 is then passed through a drying tunnel, for example, a multi-zone hot air oven, where it is dried to less than 1% by weight of residual solvent. The air temperature and velocity through the drying zone are controlled so that numerous small bubbles are generated from the solvent evaporation resulting in voids in the adhesive layer which provide the desired microporosity. The dry adhesive layer is then laminated to a web of porous backing material 32 suitably positioned so that the adhesive layer 31 is pressed into intimate contact with the porous backing material 32. Component C of the desired configuration is then die cut from the web. The silicone coated release paper is then stripped off that portion of adhesive layer 31 which overlaps flange 21 of coupling element B and film 12 of component A.

An alternative embodiment of the composite skin barrier of this invention is shown in Figure 6. In this embodiment, component C is bonded directly to polymeric film layer 12 of component A. Of course, as in the first embodiment, component C extends beyond the borders of component A so that adhesive layer 31 contacts the skin a distance from the stoma while adhesive layer 11 contacts the skin contiguous with the stoma. Coupling element B is then permanently affixed by impulse heat welding or by use of adhesives directly to porous backing layer 32 so as to encircle the starter hole 20. As shown in Figure 6, component C may extend across all of polymeric film 12 in which case a starter hole would be included to align with starter hole 20. Alternatively, component C may end directly beneath the area where flange 21 and rib 22 meet so that the skin barrier inside the coupling consists only of component A as in Figure 1.

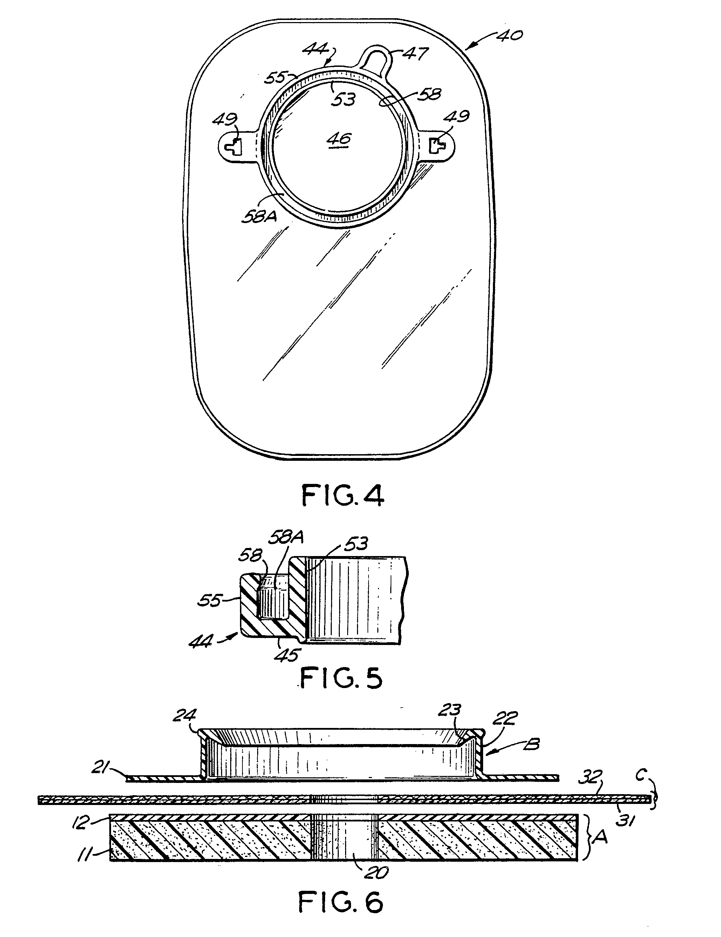

The illustrated composite skin barriers are used in conjunction with an ostomy pouch having a coupling element designed to mate with coupling element B. As shown in Figures 4 and 5, the pouch 40 includes a coupling member 44 of channel-shape seen in any radial cross-section and has a radially inner wall 53 and a radially outer wall 55. A rim 58 extends inwardly around the inner periphery of the wall 55 and, together with the wall 53, defines a restricted annular mouth or entry 58A into which, in use, the rib 22 of coupling member B is pushed to connect the pouch to the skin barrier. Rim 58 cooperates with rim 24 in providing added mechanical security. Of course, the rib 22 is dimensioned to be gripped between the channel walls. Coupling member 44 is sealed to pouch 40 around the stomal opening 46 by heat welding to surface 45. Coupling 44 is preferably of the same polymeric material as coupling B.

Figure 4 shows pouch 40 as a closed ended pouch of the type employed by most colostomates. Of course, an open ended pouch having a drainable bottom opening as employed by ileostomates or a pouch having a tap valve may also be used in conjunction with the skin barrier of this invention.

Coupling member 44 includes two ear shaped projections having openings 49 for the attachment of a belt and projection 47 which serves as a grip in uncoupling the pouch from the skin barrier.

The exposed surfaces of adhesive layer 31 and adhesive layer 11 may be covered with silicone coated release paper which is removed just prior to use.

While the rib shaped coupling 22 has been shown as component B of the skin barrier and the channel shaped coupling 44 as part of the pouch, it is possible to reverse the coupling elements. Thus, channel shaped coupling member 44 could be affixed at surface 45 to polymeric film 12 or to porous backing layer 32 in the alternative embodiment and rib shaped coupling member 22 can be affixed to the pouch by sealing flange 21 around stomal opening 46. In this case, surface 45 may be extended outwardly to function as a flange which is then overlapped by adhesive layer 31.

The coupling element B in the skin barrier of this invention has been shown as having a circular shape. However, the rib member 22 could have other configurations provided, of course, that the pouch coupling element corresponds. Also, component A has been shown as having a circular configuration and component C as having a rectangular configuration. Clearly, other geometric configurations could be employed.

Also, while the composite skin barrier of this invention has been described as consisting of three components permanently affixed to one another, it is, of course, possible to join components A and B as a single unit and attach component C separately at the time of use.

The following examples are illustrative of the invention.

Example 1

A skin barrier was prepared as follows:

Component A

An adhesive mass was prepared consisting of:

| Ingredient | Percent by weight of the adhesive layer |
|---|---|
| Polyisobutylene (Vistanex LM-MH) | 40 |
| Sodium carboxymethylcellulose | 20 |
| Pectin | 20 |
| Gelatin (powder) | 20 |

A premix was prepared by blending 2 kg. of sodium carboxymethylcellulose, 2 kg. of gelatin and 2 kg. of pectin. The blended premix was added to a heavy duty sigma blade type mixer followed by the addition of 4 kg. of polyisobutylene. Mixing was continued until the blend was homogeneous (about 25 minutes).

# 0 081 907

The resulting dough mass while hot and soft was extruded and flattened to 70 mils (1778 microns). A sheet of polyethylene of 2 mil (51 microns) thickness was laminated onto one side and silicone coated release paper on the other. The resultant mat was die cut into circular shaped wafers of about 2.5 inches (6.35 cm) in diameter having a center hole of about 0.5 inches (1.27 cm) in diameter.

Component B

A polyethylene coupling element as shown in Figures 1 to 3 was prepared by injection molding. The circular rib 22 has a diameter of about 1.75 inches (4.45 cm) and a height of about 0.18 inches (0.46 cm). The flange was affixed to the polyethylene film of component A by ultrasonic welding.

Component C

A microporous adhesive mass was prepared consisting of:

| Ingredient | Percent by weight of the microporous adhesive layer |
|---|---|
| Polyisobutylene (Vistanex L-100) | 20 |
| Polyisobutylene (Vistanex LM-MH) | 18 |
| Sodium carboxymethylcellulose | 18 |
| Gelatin (powder) | 15 |
| Terpene resin | 20 |
| Mineral oil | 8.5 |
| Butylated hydroxytoluene | 0.5 |

The above solids were dispersed in sufficient heptane to make the slurry containing 40% by weight of solids. The slurry was applied via a knife-over-roller onto silicone coated release paper to a wet thickness of 10 mil (254 microns). The material was then passed through a multi-zone oven with a residence time of 5—10 minutes so as to reduce the solvent content to less than 1%. The resulting dry adhesive layer was from about 2 to 3 mils (50.8 to 76.2 microns) thick and had a porosity of about 5 cc/sec/in$^2$ (0.775 cc/sec/cm$^2$). As the dry adhesive film emerged from the oven, it was laminated to a web of spunlaced polyester fiber (DuPont Sontara 8003) coming from a roll suitably positioned so that the adhesive was pressed into intimate contact with the spunlaced material.

The resulting microporous tape was die cut into 4 inch (10.2 cm) squares having a center hole of about 2 inches (5.1 cm). A portion of the silicone coated release paper around the center hole was stripped away and the microporous tape was pressed into contact with flange 21 of the coupling element.

The overall dimensions of components A and C and the diameter of circular rib 22 of component B were varied to obtain skin barriers usable with different size stomas.

Examples 2—11

Following the procedure of Example 1 but employing the following ingredients in adhesive layer 11 of component A other skin barriers within the scope of this invention are prepared. The ingredients are listed in percent by weight of the adhesive layer.

6

| Ingredient | 2 | 3 | 4 | 5 | 6 | 7 | 8 | 9 | 10 | 11 |
|---|---|---|---|---|---|---|---|---|---|---|
| Polyisobutylene (Vistanex LM-MH) | 40 | — | 40 | 32 | — | 30 | 50 | 45 | — | 38 |
| Polyisobutylene (Vistanex LM-MS) | — | 45 | — | — | 36 | — | — | — | 45 | — |
| Guar gum | 60 | 25 | 30 | — | — | 25 | 25 | 25 | 25 | 28.5 |
| Locust bean gum | — | — | — | — | 25 | — | — | — | — | — |
| Pectin | — | 15 | — | — | — | — | — | 15 | — | 19 |
| Karaya | — | — | — | 20 | — | — | — | — | — | — |
| Gelatin | — | — | — | 20 | — | — | — | — | — | — |
| Sodium carboxymethylcellulose | — | — | 12 | 20 | 20 | 20 | 25 | — | 15 | — |
| Cross-linked sodium carboxy-methylcellulose (Aqualon R) | — | — | 18 | — | — | 15 | — | 15 | 15 | — |
| Starch-acrylonitrile graft copolymer (Grain Processing Corp. Polymer 35-A-100) | — | 15 | — | — | — | — | — | — | — | — |
| Cross-linked dextran (Sephadex CM-C50) | — | — | — | — | 10 | — | — | — | — | — |
| Kraton 1107 | — | — | — | — | — | 10 | — | — | — | — |
| Butyl rubber (077) | — | — | — | — | 9 | — | — | — | — | 9.5 |
| Polyisobutylene (Vistanex L-100) | — | — | — | 8 | — | — | — | — | — | — |
| Cotton | — | — | — | — | — | — | — | — | — | 5 |

Examples 12—19

Following the procedure of Example 1 but employing the following ingredients in microporous adhesive layer 31 of component C other skin barriers within the scope of this invention are prepared. The ingredients are listed in percent by weight of the adhesive layer.

# 0 081 907

| Ingredient | 12 | 13 | 14 | 15 | 16 | 17 | 18 | 19 |
|---|---|---|---|---|---|---|---|---|
| Polyisobutylene (Vistanex LM-MH) | — | 20 | 25 | — | 20 | — | 25 | — |
| Polyisobutylene (Vistanex LM-LS) | 20 | — | — | 20 | — | 20 | — | 18 |
| Guar gum | 35 | 20 | — | 20 | — | 20 | 15 | 20 |
| Locust bean gum | — | — | — | — | 25 | — | — | — |
| Pectin | — | — | 10 | — | — | — | — | — |
| Karaya | — | — | — | 10 | — | — | — | — |
| Gelatin | — | — | 10 | — | — | — | — | — |
| Sodium carboxymethylcellulose | — | — | 10 | 10 | 10 | 10 | 16 | 13 |
| Cross-linked sodium carboxymethyl-cellulose (Aqualon R) | — | 15 | — | — | 10 | 10 | — | — |
| Starch-acrylonitrile graft copolymer (Grain Processing Corp. Polymer 35-A-100) | — | 18 | — | — | — | — | — | — |
| Cross-linked dextran (Sephadex CM-C50) | — | — | 7 | — | — | — | — | — |
| Terpene resin | 20 | — | 15 | 20 | 15 | 20 | 20 | 20 |
| Mineral oil | 8.5 | 8.5 | 7.5 | 8.5 | 6.5 | 6.5 | 8.5 | 8.5 |
| Butylated hydroxytoluene | 0.5 | 0.5 | 0.5 | 0.5 | 0.5 | 0.5 | 0.5 | 0.5 |
| Kraton 1107 | — | — | 15 | — | — | 13 | — | — |
| Butyl rubber (077) | — | — | — | 11 | — | — | — | — |
| Polyisobutylene (Vistanex L-100) | 16 | 18 | — | — | 13 | — | 15 | 20 |

## Claims

1. An assembly for attachment to the skin around a stoma, which comprises a first component (A) comprising an adhesive layer (11) and an upper polymeric film (12) said first component having a centrally located stomal opening (20), a second component (B) comprising a coupling element including an outwardly extending flange (21) wherein said flange is permanently affixed to the polymeric film (12) of said first component (A) in an area surrounding said stomal opening (20), and a third component (C) comprising a microporous adhesive layer (31) and an upper porous backing layer (32) said microporous adhesive layer (31) overlapping said coupling element flange (21) and polymeric film (12) and extending beyond the borders of said first component (A).

2. An assembly according to Claim 1 wherein said coupling element comprises a cylindrical rib (22) extending in an upward direction perpendicularly from said flange (21).

3. An assembly according to Claim 1 or 2 wherein the adhesive layer (11) of said first component (A) comprises a homogeneous blend of one or more pressure sensitive natural or synthetic viscous or elastomeric adhesive substances which can optionally include one or more thermoplastic elastomers and having dispersed therein one or more water soluble hydrocolloid gums which can optionally include one or more water swellable or inert natural or synthetic fibrous cohesive strengthening agents and other optional ingredients selected from antioxidants, preservatives and plasticizers. ·

4. An assembly according to Claim 3 wherein the adhesive layer (11) of said first component (A) comprises a homogeneous blend of from about 30% to about 70% by weight of a pressure sensitive substance selected from natural rubber, silicone rubber, acrylonitrile rubber, polyurethane rubber, and low molecular weight polyisobutylene and one or more optional thermoplastic elastomers selected from medium molecular weight polyisobutylene, butyl rubber, and styrene copolymers and having dispersed therein from about 35% to about 65% by weight or one or more water soluble hydrocolloids selected from sodium carboxymethyl-cellulose, pectin, gelatin, guar gum, locust bean gum, and gum karaya and one or more optional water swellable or inert cohesive strengthening agents selected from cross-linked sodium carboxymethylcellulose, starch-acrylonitrile graft copolymer, purified wood cellulose, cross-linked dextran, and cotton.

5. An assembly according to Claim 4 wherein the adhesive layer (11) of said first component (A) comprises a homogeneous blend of from about 40% to about 50% by weight of low molecular weight polyisobutylene and one or more optional thermoplastic elastomers selected from medium molecular weight polyisobutylene, butyl rubber, and styrene isoprene copolymers and having dispersed therein from about 45% to about 60% by weight of one or more water soluble hydrocolloids selected from sodium carboxymethylcellulose, pectin, gelatin, guar gum, locust bean gum, and gum karaya and one or more water swellable or inert cohesive strengthening agents selected from cross-linked sodium carboxymethyl-cellulose, starch-acrylonitrile graft copolymer, cross-linked dextran, purified wood cellulose, and cotton.

6. An assembly according to Claim 5 wherein said adhesive layer (11) consists of a blend of about 40% by weight of polyisobutylene, about 20% by weight of sodium carboxymethylcellulose, about 20% by weight of pectin, and about 20% by weight gelatin and said polymeric film (12) is polyethylene.

7. An assembly according to any preceding claim wherein said third component microporous adhesive layer (31) has a porosity of from about 1 to 100 cc/sec/in² (0.155 to 15.5 cc/sec/cm²) and comprises a homogeneous blend of one or more pressure sensitive natural or synthetic viscous or elastomeric adhesive substances which can optionally include one or more thermoplastic elastomers and having dispersed therein one or more water soluble hydrocolloid gums which can optionally include one or more water swellable cohesive strengthening agents and other optional ingredients selected from antioxidants, preservatives, plasticizers, and tackifiers and said third component porous backing layer (32) comprises woven or non-woven fabric, an open mesh polymeric substance, a polymeric foam, or a non-woven material made from polyester fibers, polypropylene fibers, nylon fibers, composite olefin fibers, or cellulose fibers.

8. An assembly according to Claim 7 wherein the microporous adhesive layer (31) of said third component comprises a homogeneous blend of from about 30% to about 60% by weight of a pressure sensitive adhesive substance selected from natural rubber, silicone rubber, acrylonitrile rubber, polyurethane rubber, and low molecular weight polyisobutylene and one or more optional thermoplastic elastomers selected from medium molecular weight polyisobutylene, butyl rubber, and styrene copolymers, from about 20% to about 65% by weight of one or more water soluble hydrocolloids selected from sodium carboxymethylcellulose, pectin, gelatin, guar gum, locust bean gum, and gum karaya and one or more optional water swellable cohesive strengthening agents selected from cross-linked sodium carboxymethylcellulose, starch-acrylonitrile graft copolymers, and cross-linked dextran, up to 10% by weight of mineral oil, and up to 25% by weight of terpene resin.

9. An assembly according to Claim 8 wherein the microporous adhesive layer (31) of said third component comprises a homogeneous blend of from about 35% to about 50% by weight of a blend of low molecular weight and medium molecular weight polyisobutylenes having dispersed therein from about 30% to about 60% by weight of one or more water soluble hydrocolloids selected from sodium carboxymethylcellulose, pectin, gelatin, guar gum, locust bean gum, and gum karaya and one or more optional water swellable cohesive strengthening agents selected from cross-linked sodium carboxymethylcellulose, starch-acrylonitrile graft copolymer, and cross-linked dextran, up to 10% by weight of mineral oil, and up to 25% by weight of terpene resin.

10. An assembly according to Claim 9 wherein said third component (C) comprises a microporous adhesive layer (31) consisting of a blend of about 18% by weight of low molecular weight polyisobutylene, about 20% by weight of medium molecular weight polyisobutylene, about 18% by weight of sodium carboxymethylcellulose, about 15% by weight of gelatin, about 20% by weight of terpene resin, about 8.5% by weight of mineral oil, and about 0.5% by weight of butylated hydroxytoluene and said porous backing (32) is spun bonded polyester fiber.

11. An assembly having the features of Claims 1, 2, 6 and 10, wherein the microporous adhesive layer (31) has a porosity of about 5 cc/sec/in² (0.775 cc/sec/cm²).

12. An assembly according to Claim 11 wherein the adhesive layer (11) of said first component (A) is about 70 mils (about 1778 microns) in thickness and said polyethylene film (12) is about 2 mils (about 50.8 microns) in thickness, and said microporous adhesive layer (31) of the third component (C) is from about 2 to about 3 mils (about 50.8 to about 76.2 microns) in thickness and said porous backing layer (32) of said third component (C) is about 10 mils (about 254 microns) in thickness.

13. An assembly for attachment to the skin around a stoma, which comprises a first component (A) comprising an adhesive layer (11) and an upper polymeric film (12) said first component having a centrally located stomal opening (20), an intermediate component (C) comprising a microporous adhesive layer (31) and an upper porous backing layer (32), said intermediate layer being bonded to and covering at least a portion of the outer polymeric film (12) of said first layer and wherein said intermediate component (C) extends beyond the borders of said first component (A), and a coupling component (B) including an outwardly extending flange (21) wherein said flange (21) is permanently affixed to said porous backing layer (32) in an area surrounding said stomal opening.

14. An assembly according to Claim 13 wherein said coupling element (13) comprises a cylindrical rib (22) extending in an upward direction perpendicularly from said flange (21).

9

**0 081 907**

15. An assembly according to Claim 13 wherein the adhesive layer (11) of said first component (A) comprises a homogeneous blend of one or more pressure sensitive natural or synthetic viscous or elastomeric adhesive substances which can optionally include one or more thermoplastic elastomers and having dispersed therein one or more water soluble hydrocolloid gums which can optionally include one or more water swellable or inert natural or synthetic fibrous cohesive strengthening agents and other optional ingredients selected from anti-oxidants, preservatives, and plasticizers.

16. An assembly according to Claim 15 wherein the adhesive layer (11) of said first component (A) comprises a homogeneous blend of from about 30% to about 70% by weight of a pressure sensitive substance selected from natural rubber, silicone rubber, acrylonitrile rubber, polyurethane rubber, and low molecular weight polyisobutylene and one or more optional thermoplastic elastomers selected from medium molecular weight polyisobutylene, butyl rubber, and styrene copolymers and having dispersed therein from about 35% to about 65% by weight of one or more water soluble hydrocolloids selected from sodium carboxymethylcellulose, pectin, gelatin, guar gum, locust bean gum, and gum karaya and one or more optional water swellable or inert cohesive strengthening agents selected from cross-linked sodium carboxymethylcellulose, starch-acrylonitrile graft copolymer, cross-linked dextran, purified wood cellulose, and cotton.

17. An assembly according to Claim 16 wherein the adhesive layer (11) of said first component (A) comprises a homogeneous blend of from about 40% to about 50% by weight of low molecular weight polyisobutylene and one or more optional thermoplastic elastomers selected from medium molecular weight polyisobutylene, butyl rubber, and styrene isoprene copolymers and having dispersed therein from about 45% to about 60% by weight of one or more water soluble hydrocolloids selected from sodium carboxymethylcellulose, pectin, gelatin, guar gum, locust bean gum, and gum karaya and one or more water swellable or inert cohesive strengthening agents selected from cross-linked sodium carboxymethylcellulose, starch-acrylonitrile graft copolymer, cross-linked dextran, purified wood cellulose, and cotton.

18. An assembly according to Claim 17 wherein said first component (A) comprises an adhesive layer (11) consisting of a blend of about 40% by weight of polyisobutylene, about 20% by weight of sodium carboxymethylcellulose, about 20% by weight of pectin, and about 20% by weight gelatin and said polymeric film (12) is polyethylene.

19. An assembly according to any one of Claims 13—18 wherein said intermediate component microporous adhesive layer (31) has a porosity of from about 1 to 100 cc/sec/in$^2$ (0.155 to 15.5 cc/sec/cm$^2$) and comprises a homogeneous blend of one or more pressure sensitive natural or synthetic viscous or elastomeric adhesive substances which can optionally include one or more thermoplastic elastomers and having dispersed therein one or more water soluble hydrocolloid gums which can optionally include one or more water swellable cohesive strengthening agents and other optional ingredients selected from antioxidants, preservatives, plasticizers, and tackifiers and said intermediate component porous backing layer (32) comprises woven or non-woven fabric, an open mesh polymeric substance, a polymeric foam, or a non-woven material made from polyester fibers, polypropylene fibers, nylon fibers, composite olefin fibers, or cellulose fibers.

20. An assembly according to Claim 19 wherein the microporous adhesive layer (31) of said intermediate component (C) comprises a homogeneous blend of from about 30% to about 60% by weight of a pressure sensitive adhesive substance selected from natural rubber, silicone rubber, acrylonitrile rubber, polyurethane rubber, and low molecular weight polyisobutylene and one or more optional thermoplastic elastomers selected from medium molecular weight polyisobutylene, butyl rubber, and styrene copolymers, from about 20% to about 65% by weight of one or more water soluble hydrocolloids selected from sodium carboxymethylcellulose, ·pectin, gelatin, guar gum, locust bean gum, and gum karaya and one or more optional water swellable cohesive strengthening agents selected from cross-linked sodium carboxymethylcellulose, starch-acrylonitrile graft copolymers, and cross-linked dextran, up to 10% by weight of mineral oil, and up to 25% by weight of terpene resin.

21. An assembly according to Claim 20 wherein the microporous adhesive layer (31) of said intermediate component (C) comprises a homogeneous blend of from about 35% to about 50% by weight of a blend of low molecular weight and medium molecular weight polyisobutylenes having dispersed therein from about 30% to about 60% by weight of one or more water soluble hydrocolloids selected from sodium carboxymethylcellulose, pectin, gelatin, guar gum, locust bean gum, and gum karaya and one or more optional water swellable cohesive strengthening agents selected from cross-linked sodium carboxymethylcellulose, starch-acrylonitrile graft copolymer, and cross-linked dextran, up to 10% by weight of mineral oil, and up to 25% by weight of terpene resin.

22. An assembly according to Claim 21 wherein said intermediate component (C) comprises a microporous adhesive layer (31) consisting of a blend of about 18% by weight of low molecular weight polyisobutylene, about 20% by weight of medium molecular weight polyisobutylene, about 18% by weight of sodium carboxymethylcellulose, about 15% by weight of gelatin, about 20% by weight of terpene resin, about 8.5% by weight of mineral oil, and about 0.5% by weight of butylated hydroxytoluene and said porous backing (32) is spun bonded polyester fiber.

10

## 0 081 907

**Patentansprüche**

1. Vorrichtung zum Befestigten auf der Haut um ein Stoma herum mit einer ersten Komponente (A) mit einer Klebschicht (11) und einer oberen Polymerschicht (12), wobei die erste Komponente in der Mitte eine Stomaöffnung (20) hat, einer zweiten Komponente (B) mit einem Verbindungsstück, das einen nach außen weisenden Flansch (21) aufweist, wobei der Flansch dauerhaft auf der Polymerschicht (12) der ersten Komponente (A) in dem die Stomaöffnung (20) umgebenden Bereich befestigt ist, und einer dritten Komponente (C) mit einer mikroporösen Klebschicht (31) und einer oberen porösen Rückseitenschicht (32), wobei die mikroporöse Klebschicht (31) den Flansch (21) des Verbindungsstückes und die Polymerschicht (12) überlappt und über die Ränder der ersten Komponente (A) herausreicht.

2. Vorrichtung nach Anspruch 1, dadurch gekennzeichnet, daß das Verbindungsstück ein zylindrisches Gestell (22) aufweist, das sich vom Flansch (21) ausgehend senkrecht nach oben erstreckt.

3. Vorrichtung nach Anspruch 1 oder 2, dadurch gekennzeichnet, daß die Klebschicht (11) der ersten Komponente (A) ein homogenes Gemisch eines oder mehrerer druckempfindlicher natürlicher oder synthetischer viskoser oder elastomerer Klebstoffe ist, das gegenbenenfalls einen oder mehrere thermoplastische Elastomere enthält, in denen ein oder mehrere wasserlösliche Hydrokolloidgummen dispergiert sind, die gegebenenfalls ein oder mehrere in Wasser quellbare oder inerte natürliche oder synthetische faserige kohäsive Verstärkungsmittel und gegebenenfalls andere Bestandteile aus der Gruppe der Antioxidantien, Konservierungsmittel und Weichmacher enthalten.

4. Vorrichtung nach Anspruch 3, dadurch gekennzeichnet, daß die Klebschicht (11) der ersten Komponente (A) ein homogenes Gemisch aus etwa 30 bis etwa 70 Gewichtsprozent eines druckempfindlichen Stoffes aus der Gruppe Naturautschuk, Silikonautschuk, Acrylnitril-Kautschuk, Polyurethan-Kautschuk und Polyisobutylen mit niedrigem Molekulargewicht und gegebenenfalls einem oder mehreren thermoplastischen Elastomeren aus der Gruppe Polyisobutylen mit mittlerem Molekulargewicht, Butylkautschuk und Styrol-Copolymerisate ist, in dem etwa 35 bis etwa 65 Gewichtsprozent ein oder mehrere wasserlöslicher Hydrokolloide aus der Gruppe Natriumcarboxymethylcellulose, Pectin, Gelatine, Guar gummi, Johannesbrotbohnengummi und Karayagummi und gegebenenfalls eines oder mehrere in Wasserquellbare oder inerte kohäsive Verstärkungsmittel aus der Gruppe vernetzter Natriumcarboxymethylcellulose, Stärke-Acrylnitril-Propfcopolymerisate, gereinigte Holzcellulose, vernetztes Dextran und Baumwolle dispergiert sind.

5. Vorrichtung nach Anspruch 4, dadurch gekennzeichnet, daß die Klebschicht (11) der ersten Komponente (A) ein homogenes Gemisch aus etwa 40 bis etwa 50 Gewichtsprozent neidermolekularem Polyisobutylen und gegebenenfalls einem oder mehreren thermoplastischen Elastomeren aus der Gruppe Polyisobutylen mittleren Molekulargewichts, Butylkautschuk und Styrol-Isopren-Copolymerisate ist, in dem etwa 45 bis etwa 60 Gewichtsprozent eines oder mehrerer wasserlöslicher Hydrokolloide aus der Gruppe Natrium-carboxymethylcellulose, Pectin, Gelatine, Guar gummi, Johannebrotbohnengummi und Karayagummi und eines oder mehrere in Wasser quellbare oder inerte kohäsive Verstärkungsmittel aus der Gruppe vernetzter Natrium-carboxymethylcellulose, Stärke-Acrylnitril-Pfropfcopolymerisate, vernetztes Dextran, gereinigte Holzcellulose und Baumwolle dispergiert sind.

6. Vorrichtung nach Anspruch 5, dadurch gekennzeichnet, daß die Klebschicht (11) aus einem Gemisch von etwa 40 Gewichtsprozent Polyisobutylen, etwa 20 Gewichtsprozent Natrium-carboxymethylcellulose, etwa 20 Gewichtzprozent Pectin und etwa 20 Gewichtsprozent Gelatine und die Polymerschicht (12) aus Polyäthylen besteht.

7. Vorrichtung nach einem der vorangehenden Ansprüche, dadurch gekennzeichnet, daß bei der dritten Komponente die mikroporöse Klebschicht (31) eine Porosität von etwa 1 bis 100 cc/sec/in$^2$ (0,155 bis 15,5 cc/sec/cm$^2$) aufweist und ein homogenes Gemisch aus einem oder mehreren druckempfindlichen natürlichen oder synthetischen viskosen oder elastomeren Klebstoffen ist, das gegebenenfalls eines oder mehrere thermoplastiche Elastomere enthält und in dem eines oder mehrere wasserlösliche Hydrokolloidgummen dispergiert sind, die gegebenenfalls eines oder mehrere wasserlösliche Hydrokolloidgummen dispergiert sind, die gegebenenfalls eines oder mehrere in Wasser quellbare kohäsive Verstärkungsmittel und gegebenenfalls andere Bestandteile aus der Gruppe der Antioxidationsmittel, Konservierungsmittel, Weichmacher und Klebrigmacher enthalten, und daß bei der dritten Komponente die poröse Rückseitenschicht (32) aus einem gewebten oder nicht gewebten Stoff, einem weitmaschigen Polymer, einem Polymerschaumstoff oder einem nicht gewebten Material aus Polyesterfasern, Polypropylenfasern, Nylonfasern, zusammengesetzten Olefinfasern oder Cellulosefasern aufgebaut ist.

8. Vorrichtung nach Anspruch 7, dadurch gekennzeichnet, daß die mikroporöse Klebschicht (31) der dritten Komponente ein homogenes Gemisch aus etwa 30 bis etwa 60 Gewichtsprozent eines druckempfindlichen Klebstoffes aus der Gruppe Naturautschuk, Silikonkautschuk, Acrylnitrilkautschuk, Polyurethankautschuk und niedermolekularem Polyisobutylen und gegebenenfalls einem oder mehreren thermoplastichem Elastomeren aus der Gruppe Polyisobutylen mittleren Molekulargewichts, Butylkautschuk und Styrol-Copolymerisaten, etwa 20 bis etwa 65 Gewichtsprozent eines oder mehrerer wasserlöslicher Hydrokolloide aus der Gruppe Natrium-carboxymethylcellulose, Pectin, Gelatine, Guargummi, Johannesbrotbohnengummi und Karayagummi sowie gegebenenfalls einem oder mehreren in Wasser quellbaren kohäsiven Verstärkungsmitteln aus der Gruppe der vernetzten Natrium-carboxymethylcellulose, Stärk-Acrylnitril-Pfropfcopolymerisate und vernetztes Dextran, bis zu 10 Gewichtsprozent Mineralöl und bis zu 25 Gewichtsprozent eines Terpenharzes ist.

9. Vorrichtung nach Anspruch 8, dadurch gekennzeichnet, daß die mikroporöse Klebschicht (31) der dritten Komponente ein homogenes Gemisch aus etwa 35 bis etwa 50 Gewichtsprozent eines Gemisches von Polyisobutylenen mit niederem Molekulargewicht und mittlerem Molekulargewicht enthält, in dem etwa 30 bis etwa 60 Gewichtsprozent eines oder mehrerer wasserlöslicher Hydrokolloide aus der Gruppe Natriumcarboxymethylcellulose, Pectin, Gelatine, Guargummi, Johannesbrotbohnengummi und Karayagummi und gegebenenfalls eines oder mehrere in Wasser quellbare kohäsive Verstärkungsmittel aus der Gruppe der vernetzten Natrium-carboxymethylcellulose, Stärke-Acrylnitril-Pfropfcopolymerisate und vernetztem Dextran, bis zu 10 Gewichtsprozent Mineralöl und bis zu 25 Gewichtsprozent eines Terpenharzes dispergiert sind.

10. Vorrichtung nach Anspruch 9, dadurch gekennzeichnet, daß die dritte Komponente (C) eine mikroporöse Klebschicht (31) aufweist, die aus einem Gemisch von etwa 18 Gewichtsprozent Polyisobutylen niedrigen Molekulargewichts, etwa 20 Gewichtsprozent Polyisobutylen mittleren Molekulargewichts, etwa 18 Gewichtsprozent Natrium-carboxymethylcellulose, etwa 15 Gewichtsprozent Gelatine, etwa 20 Gewichtsprozent Terpenharz, etwa 8,5 Gewichtsprozent Mineralöl und etwa 0,5 Gewichtsprozent butyliertem Hydroxytoluol besteht, und daß die poröse Rückseitenschicht (32) aus gesponnenen gebundenen Polyesterfasern besteht.

11. Vorrichtung nach Anspruch 1, 2, 6 und 10, dadurch gekennzeichnet, daß die mikroporöse Klebschicht (31) eine Porosität von etwa 5 cc/sec/in² (0,775 cc/sec/cm²) hat.

12. Vorrichtung nach Anspruch 11, dadurch gekennzeichnet, daß die Klebschicht (11) der ersten Komponente (A) eine Dicke von etwa 70 Mil (etwa 1778 Mikron) hat, daß die Polyäthylenschicht (12) eine Dicke von etwa 2 Mil (etwa 50,8 Mikron) hat, daß die mikroporöse Klebschicht (31) der dritten Komponente (C) etwa 2 bis etwa 3 Mil (etwa 50,8 bis etwa 76,2 Mikron) dick und die poröse Rückseitenschicht (32) der dritten Komponente (C) etwa 10 Mil (etwa 254 Mikron) dick ist.

13. Vorrichtung zum Befestigen auf der Haut im ein Stoma herum mit einer erster Komponente (A) mit einer Klebschicht (11) und einer oberen Polymerschicht (12), wobei die erste Komponente eine zentral angeordnete Stomaöffnung (20) hat, einer Zwischenkomponente (C) mit einer mikroporösen Klebschicht (31) une einer oberen porösen Rückseitenschicht (32), wobei die Zwischenschicht mit einem Teil der äußeren Polymerschicht (12) der ersten Schicht verbunden ist und sie wenigstens teilweise bedeckt und wobei die Zwischenkomponente (C) über die Ränder der ersten Komponente (A) reicht, und einer Verbindungskomponente (B) mit einem nach außen weisenden Flansch (21), wobei der Flansch (21) in einem die Stoma-Öffnung umgebenden Bereich dauerhaft an der porösen Rückseitenschicht (32) befestigt ist.

14. Vorrichtung nach Anspruch 13, dadurch gekennzeichnet, daß das Verbindungsstück (13) ein zylindrisches Gestell (22) aufweist, das vom Flansch (21) ausgehend senkrecht nach oben reicht.

15. Vorrichtung nach Anspruch 13, dadurch gekennzeichnet, daß die Klebschicht (11) der ersten Komponente (A) eine homogenes Gemisch eines oder mehrer druckempfindlicher natürlicher oder synthetischer viskoser oder elastomerer Klebstoffe ist, das gegebenenfalls einen oder mehrere thermoplastische Elastomere enthält und in dem eine oder mehrere wasserlösliche Hydrokolloidgummen dispergiert sind, die gegebenenfalls einen oder mehrere in Wasser quellbare oder inerte natürliche oder synthetische faserige kohäsive Verstärkungsmittel und gegebenenfalls andere Bestandteile aus der Gruppe der Antioxidationsmittel, Konservierungsmittel und Weichmacher enthalten.

16. Vorrichtung nach Anspruch 15, dadurch gekennzeichnet, daß die Klebschicht (11) der ersten Komponente (A) ein homogenes Gemisch von etwa 30 bis etwa 70 Gewichtsprozent einer druckempfindlichen Substanz aus der Gruppe Naturautschuk, Silikonkautschuk, Acrylnitrilkautschuk, Polyurethankautschuk und Polyisobytylen niedrigen Molekulargewichts und gegebenenfalls einem oder mehreren thermoplastischen Elastomeren aus der Gruppe Polyisobutylen mittleren Molekulargewichts, Butylkautschuk und Styrol-Copolymerisate ist, in dem etwa 35 bis etwa 65 Gewichtsprozent eines oder mehrere wasserlösliche Hydrokolloide aus der Gruppe Natrium-carboxymethylcellulose, Pectin, Gelatine, Guargummi, Johannesbrotbohnengummi und Karayagummi und gegebenenfalls eines oder mehrere in Wasser quellbare oder inerte kohäsive Verstärkungsmittel aus der Gruppe der vernetzten Natrium-Carboxymethylcellulose, Stärke-Acrylnitril-Pfropfcopolymerisate, vernetztes Dextran, gereinigte Holzcellulose und Baumwolle dispergiert sind.

17. Vorrichtung nach Anspruch 16, dadurch gekennzeichnet, daß die Klebschicht (11) der ersten Komponente (A) ein homogenes Gemisch aus etwa 40 bis etwa 50 Gewichtsprozent Polyisobutylen niedrigen Molekulargewichts und gegebenenfalls einem oder mehreren thermoplastischen Elastomeren aus der Gruppe Polyisobutylen mittleren Molekulargewichts, Butylkautschuk und Styrol-Isoprencopolymerisate ist, in dem etwa 45 bis etwa 60 Gewichtsprozent eines oder mehrere in Wasser lösliche Hydrokolloide aud der Gruppe Natriumcarboxymethylcellulose, Pectin, Gelatine, Guargummi, Johannesbrotbohnengummi und Karayagummi und eines oder mehrere in Wasser quellbare oder inerte kohäsive Verstärkungsmittel aus der Gruppe vernetzte Natrium-Carboxymethylcellulose, Stärke-Acrylnitril-Pfropfcopolymerisate, vernetztes Dextran, gereinigte Holzcellulose und Baumwolle dispergiert sind.

18. Vorrichtung nach Anspruch 17, dadurch gekennzeichnet, daß die erste Komponente (A) eine Klebschicht (11) aufweist, die aus einem Gemisch von etwa 40 Gewichtsprozent Polyisobutylen, etwa 20

12

Gewichtsprozent Natrium-carboxymethylcellulose, etwa 20 Gewichtsprozent Pectin und etwa 20 Gewichtsprozent Gelatine besteht, und daß die Polymerschicht (12) aus Polyäthylen besteht.

19. Vorrichtung nach einem der Ansprüche 13 bis 18, dadurch gekennzeichnet, daß die mikroporöse Klebschicht (31) der Zwischenkomponente eine Porosität von etwa 1 bis 100 cc/sec/in² (0,155 bis 15,5 cc/sec/cm²) hat und ein homogenes Gemisch aus einem oder mehreren druckempfindliche natürlichen oder synthetischen viskosen oder elastomeren Klebstoffen ist, das gegebenenfalls eines oder mehrere thermoplastiche Elastomere enthält und in dem eines oder mehrere in Wasser lösliche Hydrokolloidgummen dispergiert sind, die gegebenenfalls eines oder mehrere in Wasser quellbare kohäsive Verstärkungsmittel und gegebenenfalls andere Zusatzstoffe aus der Gruppe der Antioxidationsmittel, Konservierungsmittel, Weichmacher und Klebrigmacher enthalten und wobei die poröse Rückseitenschicht (32) der Zwischenkomponente aus einem gewebten oder nicht gewebten Stoff, einem weitmaschigen Polymer, einem Polymer-Schaumstoff oder einem nicht gewebten Material aus Polyesterfasern, Polypropylenfasern, Nylonfasern, zusammengesetzten Olefinfasern oder Cellulosefasern aufgebaut ist.

20. Vorrichtung nach Anspruch 19, dadurch gekennzeichnet, daß die mikroporöse Klebschicht (31) der Zwischenkomponente (C) ein homogenes Gemisch aus etwa 30 bis etwa 60 Gewichtsprozent eines druckempfindlichen Klebstoffs aus der Gruppe Naturkautschuk, Silikonkautschuk, Acrylnitrilkautschuk, Polyurethankautschuk und Polyisobutylen niedrigen Molekulargewichts und gegebenenfalls einem oder mehreren thermoplastischen Elastomeren aus der Gruppe Polyisobutylen mittleren Molekulargewichts, Bautylkautschuk und Styrol-Copolymerisaten, etwa 20 bis etwa 65 Gewichtsprozent eines oder mehrerer in Wasser löslicher Hydrokolloide aus der Gruppe Natrium-Carboxymethylcellulose, Pectin, Gelatine, Guargummi, Johannesbrotbhohnengummi und Karayagummi sowie gegebenenfalls eines oder mehrerer in Wasser quellbarer kohäsiver Verstärkungsmittel aus der Gruppe vernetzte Natrium-Carboxymethylcellulose, Stärke-Acrylnitril-Pfropfcopolymerisate und vernetztes Dextran, bis zu 10 Gewichtsprozent Mineralöl und bis zu 25 Gweichtsprozent eines Terpenharzes ist.

21. Vorrichtung nach Anspruch 20, dadurch gekennzeichnet, daß die mikroporöse Klebschicht (31) der Zwischenkomponente (C) ein homogenes Gemisch aus etwa 35 bis etwa 50 Gewichtsprozent einer Mischung von Polyisobutylenen niedrigen und mittleren Molekulargewichts ist, in dem etwa 30 bis etwa 60 Gewichtsprozent eines oder mehrerein Wasser löslicher Hydrokolloide aus der Gruppe Natrium-Carboxymethylcellulose, Pectin, Gelatine, Guargummi, Johannesbrotbohnengummi und Karayagummi und gegebenenfalls eines oder mehrerer in Wasser quellbarer kohäsiver Verstärkungsmittel aus der Gruppe vernetzte Natrium-Carboxymethylcellulose, Stärke-Acrylnitril-Pfropfcopolymerisate und vernetztes Dextran, bis zu 10 Gewichtsprozent Mineralöl und bis zu 25 Gewichsprozent Terpenharz dispergiert sind.

22. Vorrichtung nach Anspruch 21, dadurch gekennzeichnet, daß die Zwischenkomponente (C) eine mikroporöse Klebschicht (31) aufweist, die aus einem Gemisch von etwa 18 Gewichtsprozent Polyisobutylen niedrigen Molekulargewichts, etwa 20 Gewichtsprozent Polyisobutylen mittleren Molekulargewichts, etwa 18 Gewichtsprozent Natrium-Carboxymethylcellulose, etwa 15 Gewichtsprozent Gelatine, etwa 20 Gewichtsprozent Terpenharz, etwa 8,5 Gewichtsprozent Mineralöl und etwa 0,5 Gewichtsprozent butyliertes Hydroxytoluol besteht, und daß die poröse Rückseitenschicht (32) aus gesponnenen gebundenen Polyesterfasern besteht.

**Revendications**

1. Dispositif destiné à être fixé à la peau autour d'une ouverture chirurgicale artificielle, qui comprend un premier composant (A) comprenant une couche d'adhésif (11) et une pellicule polymère supérieure (12), ledit premier composant comportant une ouverture de stomie (20) placée en son centre, un second composant (B) comprenant un élément d'accouplement qui comporte un rebord (21) dirigé vers l'extérieur, ledit rebord étant fixé de maniére permanente à la pellicule polymère (12) dudit premier composant (A) dans une zone entourant ladite ouverture de stomie (20), et un troisième composant (C) comprenant une couche d'adhésif microporeux (31) et une couche dorsale poreuse supérieure (32), ladite couche d'adhésif microporeux (31) recouvrant partiellement ledit rebord (21) de l'élément d'accouplement et ladite pellicule polymère (12) et se prolongement au-delà des limites dudit premier composant (A).

2. Dispositif selon la revendication 1, dans lequel ledit élément d'accouplement comprend une nervure cylindrique (22) faisant saillie vers le haut perpendiculairement audit rebord (21).

3. Dispositif selon la revendication 1 ou 2, dans lequel la couche d'adhésif (11) dudit premier composant (A) comprend un mélange homogène d'une ou plusieurs substances adhésives visqueuses ou élastomères, naturelles ou synthétiques, sensibles à la pression, qui peuvent contenir facultativement un ou plusieurs élastomères thermoplastiques et dans laquelle ou lesquelles sont dispersées une ou plusieurs gommes hydrocolloïdes solubles dans l'eau qui peuvent contenir facultativement un ou plusieurs agents de renforcement de la cohésion, fibreux, naturels ou synthétiques, pouvant gonfler dans l'eau ou inertes, et d'autres ingrédients facultatifs choisis parmi les antioxygène, les conservateurs et les plastifiants.

4. Dispositif selon la revendication 3, dans lequel la couche d'adhésif (11) dudit premier composant (A) comprend un mélange homogène d'environ 30% à environ 70% en poids d'une substance sensible à la pression choisie entre le caoutchouc naturel, le caoutchouc de silicone, le caoutchouc d'acrylonitrile, le caoutchouc de polyuréthane, et le polyisobutylène de bas poids moléculaire et un ou plusieurs élastomères

# 0 081 907

thermoplastiques facultatifs choisis entre le polyisobutylène de poids moléculaire intermédiaire, le caoutchouc butyl, et les copolymères du styrène, et dans laquelle sont dispersés d'environ 35% à environ 65% en poids d'un ou plusieurs hydrocolloïdes solubles dans l'eau qui sont choisis entre la carboxyméthylcellulose sodique, la pectine, la gélatine, le guar, la gomme tragasol, et le karaya, et un ou plusieurs agents facultatifs de renforcement de la cohésion pouvant gonfler dans l'eau ou inertes, qui sont choisis entre la carboxyméthylcellulose sodique réticulée, un copolymère greffé d'amidon et d'acrylonitrile, la cellulose de bois purifiée, le dextranne réticulé, et le coton.

5. Dispositif selon la revendication 4, dans lequel la couche d'adhésif (11) dudit premier composant (A) comprend un mélange homogène d'environ 40% à environ 50% en poids de polyisobutylène de bas poids moléculaire et un ou plusieurs élastomères thermoplastiques facultatifs choisis entre le polyisobutylène de poids moléculaire intermédiaire, le caoutchouc butyl, et les copolymères de styrène et d'isoprène, et dans lequel sont dispersés d'environ 45% à environ 60% en poids d'un ou plusieurs hydrocolloïdes solubles dans l'eau qui sont choisis entre la carboxyméthylcellulose sodique, la pectine, la gélatine, le guar, la gomme tragasol, et le karaya, et un ou plusieurs agents de renforcement de la cohésion, pouvant gonfler dans l'eau ou inertes, qui sont choisis entre la carboxyméthylcellulose sodique réticulée, un copolymère greffé d'amidon et d'acrylonitrile, le dextranne réticulé, la cellulose de bois purifiée et le coton.

6. Dispositif selon la revendication 5, dans lequel ladite couche d'adhésif (11) se compose d'un mélange d'environ 40% en poids de polyisobutylène, d'environ 20% en poids de carboxyméthylcellulose sodique, d'environ 20% en poids de pectine, et d'environ 20% en poids de gélatine, et ladite pellicule polymère (12) est une pellicule de polyéthylène.

7. Dispositif selon l'une quelconque des revendications précédentes, dans lequel ladite couche d'adhésif microporeux (31) du troisième composant a une porosité d'environ 1 à 100 cm$^3$/seconde/pouce carré (0,155 à 15,5 cm$^3$/seconde/cm$^2$) et comprend un mélange homogène d'une ou plusieurs substances adhésives visqueuses ou élastomères, naturelles ou synthétiques, sensibles à la pression, qui peuvent facultativement contenir un ou plusieurs élastomères thermoplastiques, et dans lequel sont dispersées une ou plusieurs gommes hydrocolloïdes solubles dans l'eau qui peuvent facultativement contenir un ou plusieurs agents de renforcement de la cohésion, pouvant gonfler dans l'eau, et d'autres ingrédients facultatifs qui sont choisis entre les anti-oxygène, les conservateurs, les plastifiants et les agents d'adhérence, et ladite couche dorsale poreuse (32) du troisième composant comprend un tissu tissé ou non tissé, une substance polymère à mailles ouvertes, une mousse polymère, ou une matière non tissée faite de fibres de polyester, de fibres de polypropylène, de fibres de Nylon, de fibres d'oléfines composites, ou de fibres de cellulose.

8. Dispositif selon la revendication 7, dans lequel la couche d'adhésif microporeux (31) dudit troisième composant comprend un mélange homogène d'environ 30% à environ 60% en poids d'une substance adhésive sensible à la pression qui est choisie entre le caoutchouc naturel, le caoutchouc de silicone, le caoutchouc d'acrylonitrile, le caoutchouc de polyuréthane, et le polyisobutylène de bas poids moléculaire, et un ou plusieurs élastomères theermoplastiques facultatifs choisis entre le polyisobutylène de poids moléculaire intermédiaire, le caoutchouc butyl, et les copolymères du styrène, d'environ 20% à environ 65% en poids d'un ou plusieurs hydrocolloïdes solubles dans l'eau qui sont choisis entre la carboxyméthylcellulose sodique, la pectine, la gélatine, le guar, la gomme tragasol et le karaya, et un ou plusieurs agents facultatifs de renforcement de la cohésion, pouvant gonfler dans l'eau, qui sont choisis entre la carboxyméthylcellulose sodique réticulée, les copolymères greffés d'amidon et d'acrylonitrile, et le dextranne réticulé, jusqu'à 10% en poids d'huile minérale, et jusqu'à 25% en poids de résine de terpène.

9. Dispositif selon la revendication 8, dans lequel la couche d'adhésif microporeux (31) dudit trosième composant comprend un mélange homogène d'environ 35% à environ 50% en poids d'un mélange de polyisobutylènes de bas poids moléculaire et de poids moléculaire intermédiaire, dans lequel sont dispersés d'environ 30% à environ 60% en poids d'un ou plusieurs hydrocolloïdes solubles dans l'eau qui sont choisis entre la carboxyméthylcellulose sodique, la pectine, la gélatine, le guar, la gomme tragasol et le karaya, et un ou plusieurs agents facultatifs de renforcement de la cohésion, pouvant gonfler dans l'eau, qui sont choisis entre la carboxyméthylcellulose sodique réticulée, les copolymères greffés d'amidon et d'acrylonitrile, et le dextranne réticulé, jusqu'à 10% en poids d'huile minérale, et jusqu'à 25% en poids de résine de terpène.

10. Dispositif selon la revendication 9, dans lequel ledit troisième composant (C) comprend une couche d'adhésif microporeux (31) se composant d'un mélange d'environ 18% en poids de polyisobutylène de bas poids moléculaire, d'environ 20% en poids de polyisobutylène de poids moléculaire intermédiaire, d'environ 18% en poids de carboxyméthylcellulose sodique, d'environ 15% en poids de gélatine, d'environ 20% en poids de résine de terpéne, d'environ 8,5% en poids d'huile minérale, et d'environ 0,5% en poids d'hydroxytoluène butylé, et ladite couche dorsale poreuse (32) est faite de fibres de polyester filées.

11. Dispositif selon les revendications 1, 2, 6 et 10, dans lequel la couche d'adhésif microporeux (31) a une porosité d'environ 5 cm$^3$/seconde/pouce carré (0,775 cm$^3$/seconde/cm$^2$).

12. Dispositif selon la revendication 11, dans lequel la couche d'adhésif (11) dudit premier composant (A) a une épaisseur d'environ 70 millièmes de pouce (environ 1778 micromètres) et ladite pellicule de polyéthylène (12) a une épaisseur d'environ 2 millièmes de pouce (environ 50,8 micromètres), et ladite couche d'adhésif microporeux (31) du troisième composant (C) a une épaisseur d'environ 2 à environ 3

14

millièmes de pouce (d'environ 50,8 à environ 76,2 micromètres), et ladite couche dorsale poreuse (32) dudit troisième composant (C) a une épaisseur d'environ 10 millièmes de pouce (environ 254 micromètres).

13. Dispositif destiné à être fixé à la peau autour d'une ouverture chirurgicale artificielle, qui comprend un premier composant (A) comprenant une couche d'adhésif (11) et une pellicule polymère supérieure (12), ledit premier composant ayant une ouverture de stomie (20) placée en son centre, un composant intermédiaire (C) comprenant une couche d'adhésif microporeux (31) et une couche dorsale poreuse supérieure (32), ladite couche intermédiaire étant liée à et recouvrant au moins une partie de la pellicule polymère extérieure (12) de ladite première couche, et dans lequel ledit composant intermédiaire (C) se prolonge au-delà des limites dudit premier composant (A), et un composant d'accouplement (B) comportant un rebord (21) dirigé vers l'extérieur, ledit rebord (21) étant fixé de manière permanente à ladite couche dorsale poreuse (32) dans une zone entourant ladite ouverture de stomie.

14. Dispositif selon la revendication 13, dans lequel ledit élément d'accouplement (13) comprend une nervure cylindrique (22) faisant saillie vers le haut perpendiculairement audit rebord (21).

15. Dispositif selon la revendication 13, dans lequel la couche d'adhésif (11) dudit premier composant (A) comprend un mélange homogène d'une ou plusieurs substances adhésives visqueuses ou élastomères, naturelles ou synthétiques, sensibles à la pression, qui peuvent facultativement contenir un ou plusieurs élastomères thermoplastiques et dans lequel sont dispersées une ou plusieurs gommes hydrocolloïdes solubles dans l'eau qui peuvent facultativement contenir un ou plusieurs agents de renforcement de la cohésion, fibreux, naturels ou synthétiques, pouvant gonfler dans l'eau ou inertes, et d'autres ingrédients facultatifs choisis parmi les anti-oxygène, les conservateurs et les plastifiants.

16. Dispositif selon la revendication 15, dans lequel la couche d'adhésif (11) dudit premier composant (A) comprend un mélange homogène d'environ 30% à environ 70% en poids d'une substance sensible à la pression qui est choisie entre le caoutchouc naturel, le caoutchouc de silicone, le caoutchouc d'acrylonitrile, le caoutchouc de polyuréthane, et un polyisobutylène de bas poids moléculaire, et un ou plusieurs élastomères thermoplastiques facultatifs qui sont choisis entre le polyisobutylène de poids moléculaire intermédiaire, le caoutchouc butyl, et les copolymères du styrène, et dans lequel sont dispersés d'environ 35% à environ 65% en poids d'un ou plusieurs hydrocolloïdes solubles dans l'eau qui sont choisis entre la carboxyméthylcellulose sodique, la pectine, la gélatine, le guar, la gomme tragasol et le karaya, et un ou plusieurs agents facultatifs de renforcement de la cohésion, pouvant gonfler dans l'eau ou inertes, qui sont choisis entre la carboxyméthylcellulose sodique réticulée, les copolymères greffés d'amidon et d'acrylonitrile, le dextranne réticulé, la cellulose de bois purifiée, et le coton.

17. Dispositif selon la revendication 16, dans lequel la couche d'adhésif (11) dudit premier composant (A) comprend un mélange homogène d'environ 40% à environ 50% en poids de polyéthylène de bas poids moléculaire et un ou plusieurs élastomères thermoplastiques facultatifs choisis entre le polyisobutylène de poids moléculaire intermédiaire, le caoutchouc butyl, et les copolymères de styrène et d'isoprene, et dans lequel sont dispersés d'environ 45% à environ 60% en poids d'un ou plusieurs hydrocolloïdes solubles dans l'eau qui sont choisis entre la carboxyméthylcellulose sodique, la pectine, la gélatine, le guar, la gomme tragasol et le karaya, et un ou plusieurs agents de renforcement de la cohésion, pouvant gonfler dans l'eau ou inertes, qui sont choisis entre la carboxyméthylcellulose sodique réticulée, les copolymères greffés d'amidon et d'acrylonitrile, le dextranne purifié, la cellulose de bois purifiée et le coton.

18. Dispositif selon la revendication 17, dans lequel ledit premier composant (A) comprend une couche d'adhésif (11) se composant d'un mélange d'environ 40% en poids de polyisobutylène, d'environ 20% en poids de carboxyméthylcellulose sodique, d'environ 20% en poids de pectine, et d'environ 20% en poids de gélatine, et ladite pellicule polymère (12) est une pellicule de polyéthylène.

19. Dispositif selon l'une quelconque des revendications 13 à 18, dans lequel ladite couche d'adhésif microporeux (31) du composant intermédiaire a une porosité d'environ 1 à 100 cm$^3$/seconde/pouce carré (0,155 à 15,5 cm$^3$/seconde/cm$^2$) et comprend un mélange homogène d'une ou plusieurs substances adhésives visqueuses ou élastomères, naturelles ou synthétiques, sensibles à la pression, qui peuvent facultativement contenir un ou plusieurs élastomères thermoplastiques, et dans lequel sont dispersées une ou plusieurs gommes hydrocolloïdes solubles dans l'eau qui peuvent facultativement contenir un ou plusieurs agents de renforcement de la cohésion pouvant gonfler dans l'eau, et d'autres ingrédients facultatifs qui sont choisis entre les anti-oxygène, les conservateurs, les plastifiants et les agents d'adhérence, et ladite couche dorsale poreuse (32) du composant intermédiaire comprend un tissu tissé ou non tissé, une substance polymère à mailles ouvertes, une mousse polymère, ou une matière non tissée faite de fibres de polyester, de fibres de polypropylène, de fibres de Nylon, de fibres d'oléfines composites, ou de fibres de cellulose.

20. Dispositif selon la revendication 19, dans lequel la couche d'adhésif microporeux (31) dudit composant intermédiaire (C) comprend un mélange homogène d'environ 30% à environ 60% en poids d'une substance adhésive sensible à la pression qui est choisie entre le caoutchouc naturel, le caoutchouc de silicone, le caoutchouc d'acrylonitrile, le caoutchouc de polyuréthane, et le polyisobutylène de bas poids moléculaire, et un ou plusieurs élastomères thermoplastiques facultatifs qui sont choisis entre le polyisobutylène de poids moléculaire intermédiaire, le caoutchouc butyl est les copolymères du styrène, d'environ 20% à environ 65% en poids d'un ou plusieurs hydrocolloïdes solubles dans l'eau qui sont choisis entre la carboxyméthylcellulose sodique, la pectine, la gélatine, le guar, la gomme tragasol et le karaya, et un ou plusieurs agents facultatifs de renforcement de la cohésion, pouvant gonfler dans l'eau, qui sont choisis entre la carboxyméthylcellulose sodique réticulée, les copolymères greffés d'amidon et d'acrylonitrile et le dextranne réticulé, jusqu'à 10% en poids d'huile minérale, et jusqu'à 25% en poids de résine de terpène.

21. Dispositif selon la revendication 20, dans lequel la couche d'adhésif microporeux (31) dudit composant intermédiaire (C) comprend un mélange homogène d'environ 35% à environ 50% en poids d'un mélange de polyisobutylènes de bas poids moléculaire et de poids moléculaire intermédiaire dans lequel sont dispersés d'environ 30% à environ 60% en poids d'un ou plusieurs hydrocolloïdes solubles dans l'eau qui sont choisis entre la carboxyméthylcellulose sodique, la pectine, la gélatine, le guar, la gomme tragasol et le karaya, et un ou plusieurs agents facultatifs de renforcement de la cohésion, pouvant gonfler dans l'eau, qui sont choisis entre la carboxyméthylcellulose sodique réticulée, les copolymères greffés d'amidon et d'acrylonitrile et le dextranne réticulé, jusqu'à 10% en poids d'huile minérale, et jusqu'à 25% en poids de résine de terpène.

22. Dispositif selon la revendication 21, dans lequel ledit composant intermédiaire (C) comprend une couche d'adhésif microporeux (31) se composant d'un mélange d'environ 18% en poids de polyisobutylène de bas poids moléculaire, d'environ 20% en poids de polyisobutylène de poids moléculaire intermédiaire, d'environ 18% en poids de carboxyméthylcellulose sodique, d'environ 15% en poids de gélatine, d'environ 20% en poids de résine de terpène, d'environ 8,5% en poids d'huile minérale, et d'environ 0,5% en poids d'hydroxytoluène butylé, et ladite couche dorsale poreuse (32) est faite de fibres de polyester filées.

FIG. 1

FIG. 2

FIG. 3

1

0 081 907

FIG. 4

FIG. 5

FIG. 6

2